# EUROPEAN PATENT SPECIFICATION

(11) **EP 3 106 523 B1**
(45) Date of publication and mention of the grant of the patent: **23.06.2021**
(21) Application number: 15746820.8
(22) Date of filing: 09.02.2015
(51) Int. Cl.: C12P 7/22, C12P 7/26, C12R 1/01

(54) **METHOD FOR PRODUCING PHENYL PROPANE-BASED COMPOUND USING MICRO-ORGANISMS**
VERFAHREN ZUR HERSTELLUNG VON PHENYLPROPANBASIERTER VERBINDUNG MIT MIKROORGANISMEN
PROCÉDÉ DE PRODUCTION D'UN COMPOSÉ À BASE DE PHÉNYLPROPANE FAISANT APPEL À DES MICRO-ORGANISMES

(30) Priority: 10.02.2014 JP 2014023839
(43) Date of publication of application: 21.12.2016
(73) Proprietor: Japan Agency for Marine-Earth Science and Technology, Yokosuka-shi, Kanagawa 237-0061 (JP)
(72) Inventor: OHTA, Yukari, Yokosuka-shi Kanagawa 237-0061 (JP); HATADA, Yuji, Yokosuka-shi Kanagawa 237-0061 (JP); HASEGAWA, Ryoichi, Hyogo 6660035 (JP)
(74) Representative: Schulz Junghans Patentanwälte PartGmbB
(86) International application number: PCT/JP2015/053547
(87) International publication number: WO 2015/119276

(56) References cited:
- EP-A1- 2 218 773
- JP-A- 2002 034 557
- JP-A- 2006 230 255
- JP-A- 2012 130 326
- US-A1- 2012 196 334
- Y. OHTA ET AL: "Screening and Phylogenetic Analysis of Deep-Sea Bacteria capable of Metabolizing Lignin-Derived Aromatic Compounds", OPEN JOURNAL OF MARINE SCIENCE, vol. 2, 31 October 2012 (2012-10-31), pages 177-187, XP002773594, DOI: 10.4236/ojms.2012.24021
- YUKARI OHTA ET AL: "Draft Genome Sequence of Novosphingobium sp. Strain MBES04, Isolated from Sunken Wood from Suruga Bay, Japan", GENOME ANNOUNCEMENTS, vol. 3, no. 1, E01373-1, 15 January 2015 (2015-01-15), pages 1-2, XP055218472, US ISSN: 2169-8287, DOI: 10.1128/genomeA.01373-14
- GALL, D.L. ET AL.: 'Stereochemical Features of Glutathione-dependent Enzymes in the Sphingobium sp. Strain SYK-6 beta-Aryl Etherase Pathway' THE JOURNAL OF BIOLOGICAL CHEMISTRY vol. 289, no. 12, pages 8656 - 8667, XP055218446
- AKIHITO OCHIAI ET AL.: 'Sphingomonas wa Yutosei' JOURNAL OF THE SOCIETY FOR BIOSCIENCE AND BIOENGINEERING, JAPAN vol. 89, no. 10, October 2011, page 614, XP008182268
- TAKAO KISHIMOTO: 'Synthesis of Lignin Model Compounds and Their Application to Wood Research' JOURNAL OF WOOD SCIENCE vol. 55, no. 4, 2009, pages 187 - 197, XP055218459
- MASAI, E. ET AL.: 'Complete Genome Sequence of Sphingobium sp. Strain SYK-6, a Degrader of Lignin-Derived Biaryls and Monoaryls' JOURNAL OF BACTERIOLOGY vol. 194, no. 2, January 2012, pages 534 - 535, XP055218448
- YUKARI OTA ET AL.: 'Shinkai Chinboku kara Tanri shita Bacteria no Bunruigakuteki Ichi to Hokozoku Monomer Taisha' DAI 57 KAI PROCEEDINGS OF THE LIGNIN SYMPOSIUM 01 October 2012, page 128, XP008182505
- GALL, D.L. ET AL.: 'A Group of Sequence-Related Sphingomonad Enzymes Catalyzes Cleavage of beta- Aryl Ether Linkages in Lignin beta-Guaiacyl and beta- Syringyl Ether Dimers' ENVIRON. SCI. TECHNOL. vol. 48, September 2014, pages 12454 - 12463, XP055218461
- KAGAMI IIDA ET AL.: 'Shinkai Chinboku kara Tanri shita Novosphingobium-zoku Saikin no 2 Ryotai Lignin Model Kagobutsu beta-0-4 Ketsugo Kangenteki Kairetsu Kosogun no Kaiseki' DAI 64 KAI ABSTRACTS OF THE ANNUAL MEETING OF THE JAPAN WOOD RESEARCH SOCIETY ( CD VER. 03 March 2014, XP008182386
- KANAKO KUROSAWA ET AL.: 'Shinkai Yurai Novosphingobium-zoku Saikin o Mochiita Biomass kara no Hokozoku Monomer Seisan' DAI 64 KAI ABSTRACTS OF THE ANNUAL MEETING OF THE JAPAN WOOD RESEARCH SOCIETY 03 March 2014, XP008182384
- YUKARI OTA ET AL.: 'Shinkai Chinboku kara Tanri shita Biseibutsu ga Seisan suru Lignin Yurai Hokozoku Monomer Genryo to shite no Potential' DAI 64 KAI ABSTRACTS OF THE ANNUAL MEETING OF THE JAPAN WOOD RESEARCH SOCIETY ( CD VER. 03 March 2014,
- OHTA, Y. ET AL.: 'Draft Genome Sequence of Novosphingobium sp. Strain MBES04, Isolated from Sunken Wood from Suruga Bay, Japan' GENOME ANNOUNCEMENTS vol. 3, no. ISSUE, 15 January 2015, pages E01373 - 14, XP055218472
- None

## Description

This invention relates to a method for specifically producing a compound having a phenyl propane structure from biomass containing lignins by using microorganisms.

### Background Art

Lignins are amorphous polymeric substances existing in plants as ingredients of vascular bundle cell walls. Lignins are formed as a result of complex condensation reactions of phenyl propane-based constituent units and show a remarkable chemical structural characteristic of containing methoxy groups. Lignins take a role of causing lignified plant cells to mutually agglutinate, thereby strengthening plant tissues. Lignins are contained by 18 to 36% in woods and by 15 to 25% in herbaceous plants. Thus, various attempts have been and being made to degrade lignins and obtain useful compounds therefrom for the purpose of effectively exploiting wood resources.

Meanwhile, known phenyl propane-based compounds include coumaric acid, cinnamic acid, caffeic acid (3,4-dihydorxy-cinnamic acid), eugenol, anethol, coniferyl alcohol, sinapyl alcohol and ferulic acid. In industrial fields, phenyl propane-based compounds are useful because such compounds can be used for medicines, functional foods and synthetic intermediates of various chemical products such as perfumes, spices, essential oils, fungicides, anesthetics and antioxidant agents.

For example, methods for non-specifically degrading lignins contained in lignocellulose substances of woods, rice straws and so on down to low molecules by using physiochemical techniques such as gasifying techniques involving high-temperature high-pressure processing (see Patent Literatures 1 and 2 listed below) and pressurized hot water treatment techniques (see Patent Literature 3 listed below) are known. However, with any of the above listed techniques, it is very difficult to produce a specific compound from lignins. In other words, when any of the above listed techniques is employed, phenyl propane-based compounds having three carbon atoms in each carbon side chain that is directly bonded to a benzene ring skeleton, which compounds operate as unit structures of lignins, are further degraded to lower molecules . To be more accurate, phenyl propane-based compounds are non-specifically transformed into phenols such as guaiacols, sylingols and so on where one or more than one carbon side chains having no carbon atoms are directly bonded to a benzene ring skeleton and also into phenyl methane compounds such as vanillin, syringaldehyde and so on where one or more than one carbon side chains having one carbon atoms are directly bonded to a benzene ring skeleton. Additionally, degradation products of lignins are mixtures of a variety of components and hence it is highly difficult to obtain only phenyl propane-based compounds therefrom. In other words, it is not possible to specifically produce phenyl propane-based compounds with any of the above identified techniques. Furthermore, if any of the above listed physiochemical methods is adopted and put to practical use, it requires a vast amount of energy and special apparatus.

In view of the above-identified problems, attempts are being made to obtain degradation products of lignins by biologically processing lignins. For example, a method of degrading lignins by inoculating white-rot fungus into lignocellulose substances and incubating them there is known (see Patent Literature 4 listed below).

Meanwhile, since it is known that *β*-aryl ether type bonds account for about 50% of the chemical bonds existing in natural lignins, if *β*-aryl ether bonds can be cleaved or not is highly significant from the viewpoint of degrading natural lignins. Known microorganisms that produce enzymes capable of specifically cleaving *β*-aryl ether type bonds include microorganisms of the genus Sphingobium (see Patent Literature 5 and Non-Patent Literature 1 listed below; note, however, microorganisms of that genus are described as those the genus Pseudomonas in Non-Patent Literature 1), microorganisms of the genus Brevundimonas (see Patent Literature 6) and microorganisms of the genus Pseudomonas (see Non-Patent Literature 2 listed below) . Ohta et al. (Open Journal of Marine Science, Vol. 2, 2012, pp. 177-187) relates to a screen of deep-sea bacteria identified several bacterial species capable of metabolizing lignin model compounds, among them Novosphingobium mathurense. US 2012-0196334 A1 discloses the use of enzymes isolated from Novosphingobium aromaticivorans in degradation of lignin to valuable aromatic chemicals.

Patent Literatures 4 through 6 and Non-Patent Literatures 1 and 2 describe microorganisms of the genus Sphingobium, microorganisms of the genus Brevundimonas and microorganisms of the genus Pseudomonas as well as methods of producing phenyl propane-based compounds by cleaving *β*-aryl ether type bonds of guaiacylglycerol-*β*-guaiacyl ether or 3-hydroxy-2-(2-methoxyphenoxy)-1-(3-methoxy-4-hydroxypheny 1)-1-propanone, which are model compounds of lignins, by means of enzymes capable of cleaving *β*-aryl ether type bonds that are produced by microorganisms of any of the above listed genera.

### [Prior Art Literatures]

### [Patent Literatures]

Patent Literature 1: JP 2012-140346 A
Patent Literature 2: JP 2012-50924 A
Patent Literature 3: JP 2010-239913 A
Patent Literature 4: JP 1975-46903 A
Patent Literature 5: JP 1993-336976 A
Patent Literature 6: JP 2002-34557 A

### [Non-Patent Literatures]

Non-Patent Literature 1: FEBS Lett. 249 (2), 1989, pp. 348-352
Non-Patent Literature 2: Mokuzai Gakkaishi, Vol. 31 (11), 1985, pp. 956-958
Non-Patent Literature 3: Biosci Biotechnol Biochem. 2011; 75 (12):2404-7

### [Disclosure of the Invention]

### [Problem to be solved by the Invention]

According to the description of Patent Literature 4, the method described in the literature can provide possibility of degrading lignins by causing microorganisms to act on the lignins to be degraded. However, when white-rot fungi and ligninase that white-rot fungi produce are caused to act on lignocellulose substances, not only the products lack structural uniformity but also polymerization reactions mainly of the products progress because the specificity of the lignin degrading reaction caused by the microorganisms is very low. Therefore, the method described in Patent Literature 4 has a problem that the reaction products lack usefulness as industrial raw materials.

The methods described in Patent Literatures 5 and 6 and Non-Patent Literatures 1 through 3 utilize microorganisms and enzymes produced by microorganisms of the type under consideration to produce phenyl propane-based compounds from model compounds of lignin. However, these literatures do not describe any fact that phenyl propane-based compounds are obtained from natural biomass by causing microorganisms and enzymes to act on model compounds of lignin. In particular, it is not possible to produce phenyl propane-based compounds only by using the enzymes described in Patent Literature 5. It is not possible either to produce phenyl propane-based compounds by using the microorganisms described in Non-Patent Literature 1 because those microorganisms completely break down guaiacylglycerol-*β*-guaiacyl ether down to CO2 for anabolism.

While microorganisms described in Non-Patent Literature 2 are capable of metabolizing guaiacylglycerol-*β*-guaiacyl ether, the yield of the obtained product is very low because the produced phenyl propane-based compounds are further metabolized and transformed into different compounds.

Therefore, the problem to be solved by the invention is to provide a method which, if compared with the methods described in the prior art literature, more specifically and efficiently produces a compound having a phenyl propane structure from natural biomass containing lignins by causing microorganisms to act on the biomass.

### [Means for Solving the Problem]

The inventors of the present invention have paid intensive research efforts to solve the above identified problem, conducting operations of screening microorganisms obtained from sunken woods in deep seas and capable of degrading lignin-related compounds. As a result, the inventors of the present invention succeeded in isolating a novel strain of microorganisms that degrade lignin-related compounds from more than 1,000 strains of microorganisms. Furthermore, not only microorganisms of the strain can produce phenyl propane-based compounds from model compounds of lignins but also they can specifically produce phenyl propane-based compounds, using natural lignin-containing biomass as substrates. Additionally, the inventors of the present invention found that microorganisms of the strain can efficiently degrade model compounds of lignins and produce phenyl propane-based compounds at a high yield. The present invention is based on these successful researches and findings.

Thus, the present invention provides a method for producing a phenyl propane-based compound comprising a step of producing a phenyl propane-based compound by causing the microorganism Novosphingobium species MBES04 (accession number: NITE P-01797) to act on biomass containing lignins.

Preferably, in the production method of the present invention, the phenyl propane-based compound is at least one selected from a group of 3-hydroxy-1-(4-hydroxy-3-methoxyphenyl)-1-propanone, 3-hydroxy-1-(4-hydroxy-3,5-dimethoxyphenyl)-1-propanone and 3-hydroxy-1-(4-hydroxyphenyl)-1-propanone.

### [Advantages of the Invention]

Thus, with the production method of the present invention and microorganisms provided herein, it is possible to specifically and efficiently produce a compound having a phenyl propane structure from biomass containing lignins originating from natural materials such as agricultural wastes and woods.

### [Brief Description of the Drawings]

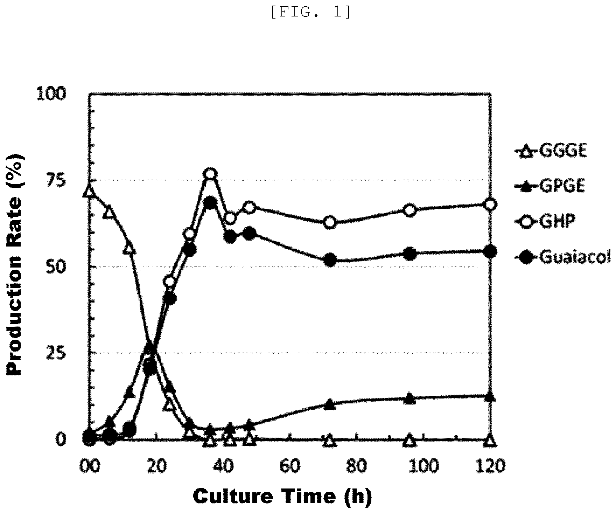

FIG. 1 is a graph illustrating the change with time of the quantity of a substrate and also the quantity of the reaction product obtained by causing guaiacylglycerol-*β*-guaiacyl ether to act on MBES04 strain. In the graph, GGGE represents guaiacylglycerol-*β*-guaiacyl ether and GPGE represents 1-(4-hydroxy-3-methoxyphenyl)-3-hydroxy-2-(2-methoxyphenox y)propane-1-on, while GHP represents 3-hydroxy-1-(4-hydroxy-3-methoxyphenyl)-1-propanone and Guaiacol simply means guaiacol.

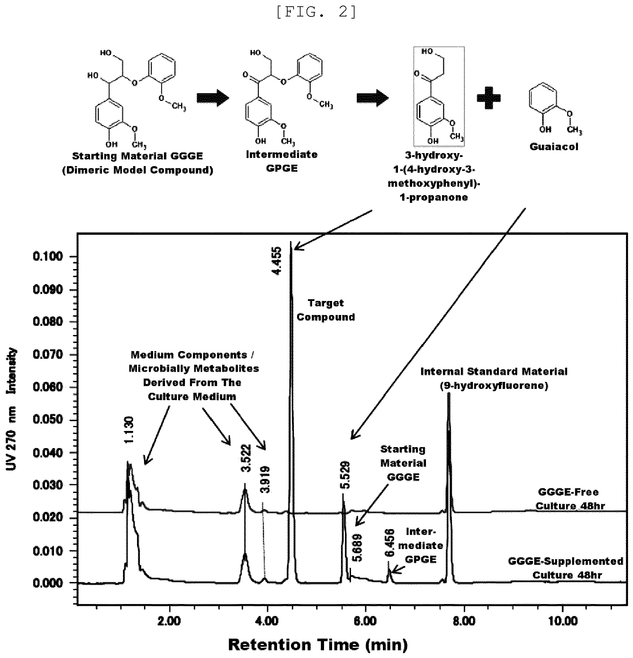

FIG. 2 is a schematic illustration of the chromatogram of the culture supernatant obtained by causing guaiacylglycerol-*β*-guaiacyl ether to act on MBES04 strain. In the graph, the vertical axis (UV 270 nm Intensity) indicates the intensity of UV rays at the wavelength of 270 nm.

### [Best Mode for Carrying Out the Invention]

Now, the present invention will be described in greater detail below.

The production method of the present invention is a method of producing a phenyl propane-based compound comprising a step of obtaining a phenyl propane-based compound by causing microorganisms of the genus Novosphingobium to act on biomass containing lignins.

With the production method of the present invention, phenyl propane-based compounds such as 3-hydroxy-1-(4-hydroxy-3-methoxyphenyl)-1-propanone, 3-hydroxy-1-(4-hydroxy-3,5-dimethoxyphenyl)-1-propanone and 3-hydroxy-1-(4-hydroxyphenyl)-1-propanone are produced as microorganisms of the genus Novosphingobium are caused to exert metabolic effects on lignins in biomass.

The phenyl propane-based compound that can be obtained by the above defined production method of the present invention is a compound expressed by general formula (A) shown below: 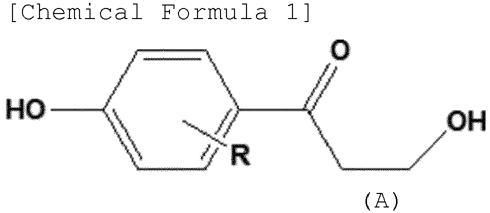 (where R represents 1 or 2 or more than 2 alkyl groups or alkoxy groups having 1 to 5 carbon atoms or hydrogen atoms) that has a carbonyl group at 1-position and hydroxyl groups respectively at 3-position and at 4-position of a phenyl group. More specifically, the compound is 3-hydroxy-1-(4-hydroxy-3-methoxyphenyl)-1-propanone, 3-hydroxy-1-(4-hydroxy-3,5-dimethoxyphenyl)-1-propanone or 3-hydroxy-1-(4-hydroxyphenyl)-1-propanone and, more preferably, it is 3-hydroxy-1-(4-hydroxy-3-methoxyphenyl)-1-propanone.

Of the above listed compounds, for example, 3-hydroxy-1-(4-hydroxy-3-methoxyphenyl)-1-propanone has a structure expressed by formula (I) shown below.

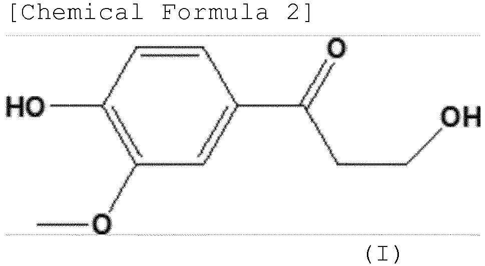

3-hydroxy-1-(4-hydroxy-3-methoxyphenyl)-1-propanone can qualitatively and quantitatively be analyzed, for example, by means of reverse phase HPLC. The conditions to be met for reverse phase HPLC include that an Octa Decyl Silyl group-modified silica gel column (ODS column) is to be employed along with eluent A (2mM ammonium acetate, 0.05%V/V formic acid) and eluent B (100%V/V methanol), that a column temperature of 40°C and a flow rate of 1.2 ml/min are to be set and that a mixture solution of eluent A 90%V/V and eluent B 10%V/V is to be fed for a minute and subsequently eluent B is to be fed at a gradient of 10%V/V to 95%V/V for seven minutes. As the above conditions are satisfied, 3-hydroxy-1-(4-hydroxy-3-methoxyphenyl)-1-propanone can be detected with a peak of retention time of around 4. 5 minutes by using a UV detector (270nm) . It can be quantified by means of a calibration curve method, an internal standard method or the like provided that standard 3-hydroxy-1-(4-hydroxy-3-methoxyphenyl)-1-propanone is employed.

The above defined production method of the present invention employs biomass containing lignins. While lignins to be used as starting material are not subjected to any particular limitations so long as they are known to those who are skilled in the art, they are typically found in vascular bundles of plants and known to have a complicatedly polymerized dendritic structure where 3 types of phenylpropanoid that are expressed respectively by chemical formulas (B) through (D) shown below operate as unit constituents.

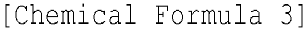

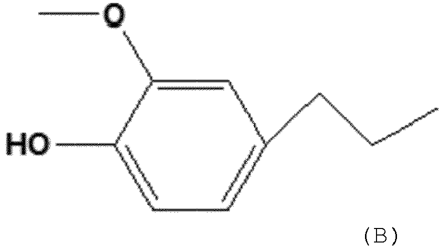

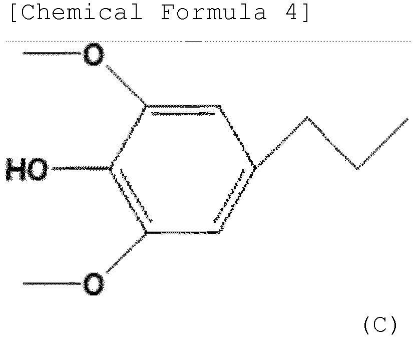

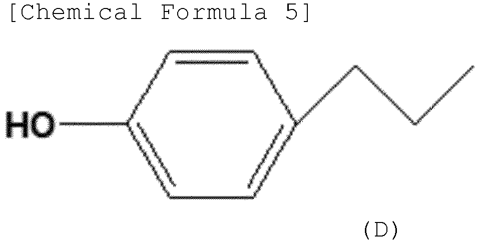

Lignin-related substances are not subjected to any particular limitations so long as they are substances derived from lignins . As far as this specification is concerned, lignin-related substances include substances that are regarded as model compounds of lignin such as guaiacylglycerol-*β*-guaiacyl ether in addition to degradation products of lignins and substances that can be obtained by processing lignins. As far as this specification is concerned, biomass is not subjected to any particular limitations so long as it contains lignins and/or lignin-related substances. For example, biomass includes natural products such as grasses and trees, substances that can be obtained by processing grasses and trees and agricultural wastes.

Biomass containing lignins and lignin-related substances (which may also be referred to as lignin-containing biomass hereinafter) can take any of the forms of, for example, solid, suspension and liquid and the forms that biomass can take vary depending on if it is preprocessed or not. For example, lignin-containing biomass can take the form of suspension obtained by crushing the biomass and adding liquid to the crushed biomass.

Lignin-containing biomass may be extracted lignins. Examples of extracted lignins include extracted liquid lignins obtained by powdering lignin-containing biomass, preparing a suspension by causing the powdered lignin-containing biomass to be suspended in a solvent that is suited for extraction of lignins so as to produce a suspension of between 0.1%W/V and 50%W/V, preferably between 1%W/V and 20%W/V, subjecting the suspension to an extraction process at temperatures between 10°C and 150°C, preferably between 20°C and 130°C, more preferably between 20°C and 80°C, for a period between several hours and several days, preferably between an hour and 6 days, and subsequently removing the solid contents from the extraction-processed solution and extracted solid lignins obtained by removing the solvent from the extracted liquid lignins and drying the evaporation residue.

Solvents that can be used for the purpose of the present invention and are suited for extraction of lignins are not subjected to any particular limiations. Examples of such solvents include water, dioxane, low molecular weight alcohols such as methanol and isopropanol, dimethyl formaldehyde and so on, of which water and dioxane are preferable.

With the above defined production method of the present invention, enzymes derived from Novosphingobium species MBES04 (to be also referred to as MBES04 strain hereinafter) are employed in order to obtain phenyl propane-based compounds from lignin-containing biomass.

The mycological properties, the physiological properties and the utilization potential as substrate of MBES04 strain will be described hereinafter in Examples 3 and 4. The base sequence of the DNA that encodes 16S ribosome RNA of MBES04 strain is described in the sequence listing with the sequence number 3 (accession number: AB733576). In view of these descriptions, therefore, microorganisms of the genus Novosphingobium that have the mycological properties, the physiological properties and the utilization potential as substrate or the base sequence of MBES04 strain can be regarded as those that correspond to or are equivalent to microorganisms of MBES04 strain. Particularly, microorganisms of the genus Novosphingobium that show agreement with the enzyme activity described in Table 1 below by not less than 90%, preferably not less than 95%, more preferably not less than 99%, agreement with the utilization potential as substrate described in Table 2 shown below by not less than 90%, preferably not less than 95%, more preferably not less than 99% and/or identity with the base sequence described in the sequence listing with the sequence number 3 by not less than 98%, preferably not less than 99%, more preferably not less than 99.5% can be regarded substantially as those of MBES04 strain. As far as this specification is concerned, the expression of "identity" of sequences means strict identity of two sequences that is observed when the two sequences are aligned with each other and does not mean similarity between the two sequences.

Likewise, microorganisms of the genus Novosphingobium to be used for the production method of the present invention can be isolated from woods or the like in the realm of nature where, for example, corrosion and rottenness are observed, on the basis of the mycological properties, the physiological properties and the utilization potential as substrate and/or the base sequence of MBES04 strain. While there are no particular limitations to the method for isolating microorganisms of MBES04 strain, purely cultured microorganisms of the strain can be obtained, for example, by means of a streak culture method and a limiting dilution method.

Phenyl propane-based compounds can be obtained by causing microorganisms of the genus Novosphingobium to act on lignin-containing biomass. Note that the expression of "causing microorganisms of the genus Novosphingobium to act on" as used herein means causing microorganisms of the genus Novosphingobium to exert the potential ability they have of transforming lignins and lignin-related substances into phenyl propane-based compounds.

There are no particular limitations to the conditions of a system (to be referred to as action system hereinafter) for causing lignin-containing biomass to act on microorganisms of the genus Novosphingobium so long microorganisms of the genus Novosphingobium can survive under the conditions. For example, they are the conditions under which microorganisms of the genus Novosphingobium can be cultured. When microorganisms of the genus Novosphingobium are those of MBES04 strain, for example, the conditions under which such microorganisms can be cultured include temperatures between 10 and 45°C, preferably between 15 and 37°C, pH values between 5.5 and 8.5, preferably between 6 and 8 and NaCl concentrations between 0 and 6%W/V, preferably between 0 and 3%W/V.

Additionally, culture medium ingredients may be allowed to exist in such an action system. There are no particular limitations to culture medium ingredients that can be used for the purpose of the present invention so long as they are normally known culture medium ingredients for microorganisms. Such culture medium ingredients typically include carbon sources, nitrogen sources, inorganic substances and micronutrients that may be required for the microorganisms strain to be used. These ingredients may be either natural substances or synthesized substances.

There are no particular limitations to carbon sources to be used for the purpose of the present invention so long as they have utilization potential for microorganisms of the genus Novosphingobium. Examples of carbon sources include carbohydrates such as glucose, lactose, maltose, cellobiose, xylose and dextrin, organic acids such as gluconic acid, pyruvic acid and succinic acid, amino acids such as alanine and serine and aromatic hydrocarbons such as vanillin, ferulic acid and benzoic acid. Any one of the above-listed substances or a combination of two or more than two of them may be used as carbon source for the purpose of the present invention.

There are no particular limitations to nitrogen sources to be used for the purpose of the present invention so long as they have utilization potential for microorganisms of the genus Novosphingobium. Examples of nitrogen sources include organic nitrogen sources such as peptone, polypeptone, bacto peptone, meat extracts, yeast extracts, corn steep liquor, soybean flour, soybean cake and yeast extracts and inorganic nitrogen sources such as ammonium chloride, ammonium sulfate, urea, ammonium nitrate, sodium nitrate and ammonium phosphate. Any one of the above listed substances or a combination of two or more than two of them may be used as nitrogen source for the purpose of the present invention.

Examples of inorganic substances that can be used for the purpose of the present invention include calcium salts, magnesium salts, potassium salts, sodium salts, phosphates, manganese salts, zinc salts, iron salts, copper salts, molybdenum salts and cobalt salts. More specifically, examples of inorganic substances include potassium dihydrogenphosphate, dipotassium hydrogenphosphate magnesium sulfate, iron(I) sulfate, manganese sulfate, zinc sulfate, sodium chloride, potassium chloride and calcium chloride. Any one of the above listed substances or a combination of two or more than two of them may be used for the purpose of the present invention. Additionally, examples of micronutrients that can be used for the purpose of the present invention include amino acids and micronutrients vitamins such as biotin and thiamine.

There are no particular limitations to the concentrations of lignins, lignin-related substances and culture medium ingredients and also to the quantities of various ingredients such as the number of microorganisms of the genus Novosphingobium in an action system to be used for the purpose of the present invention. In other words, such concentrations and quantities can appropriately be selected. Additionally, it is preferable to agitate and shake the action system in order to raise the frequency of contact between lignins and lignin-related substances and microorganisms of the genus Novosphingobium. There are no particular limitations to the duration of any action in the action system so long as production of one or more than one phenyl propane-based compounds is recognized in the action system. Typical duration of an action is between several hours and several days and the duration can appropriately be selected as a function of the culturing method to be adopted for the action system. When a batch culture method is adopted, the duration of an action is preferably between about 12 hours and 36 hours or more than 36 hours.

The phenyl propane-based compound or compounds produced in an action system can be used as they are without requiring any particular treatment. However, the produced phenyl propane-based compounds are preferably brought into a roughly refined state by isolating them from microorganisms of the genus Novosphingobium after the end of the action. Additionally, the produced phenyl propane-based compounds can be refined by using a normally known means for refining aromatic compounds such as a solid phase extraction method or a chromatogram method. Furthermore, the phenyl propane-based compounds can be obtained as solid by evaporating the solvent and drying the compounds.

As far as the object of the present invention is achievable, various steps and operations may be added to the production method of the present invention before and/or after the above defined steps.

Now, a specific exemplar mode of carrying out the production method of the present invention will be described below.

Lignin-containing biomass is suspended in water or dioxane and the suspension is kept warm at temperatures between 20 and 80°C in a water bath for a period between an hour and ten hours. After the temperature keeping period, the solid components are removed from the suspension to obtain solution of extracted lignins. Then, an alkali compound is dropped to the lignin-extract solution to a small extent to adjust the pH value and make the solution weakly acidic. Subsequently, nutrient culture medium ingredients and a magnesium salt are added to the lignin extract solution. Then, the obtained solution is sterilized and microorganisms of the genus Novosphingobium are inoculated into the sterilized solution. After culturing the microorganisms at temperatures between 15 and 40°C for a period between 10 and 120 hours, the obtained culture solution is subjected to a centrifugal operation to obtain culture supernatant. The target compounds, which are phenyl propane-based compounds, are refined from the culture supernatant and then dried and solidified in inert gas to obtain solid phenyl propane-based compounds.

The phenyl propane-based compounds obtained by the production method of the present invention can be utilized as starting materials or intermediates for producing resins, adhesive agents, resist materials and drugs. More specifically, when 3-hydroxy-1-(4-hydroxy-3-methoxyphenyl)-1-propanone is obtained by the production method of the present invention, the compound can be transformed into coniferyl alcohol and so on that are industrially useful as starting materials of drugs, perfumes, food materials and so on by way of 1-(4-hydroxy-3-methoxyphenyl)-1,3-propanediol.

Microorganisms that can be used for the production method of the present invention are those of Novosphingobium sp. MBES04 (accession number: NITE P-01797). MBES04 strain is expressed as Novosphingobium sp. MBES04 for microorganism identification and was deposited in the Fermentation Res. Inst. Microorganism Depository Center (T̅292-0818 2-5-8 Kazusakamatari, Kisarazu City, Chiba Prefecture) of the National Institute of Technology and Evaluation (NITE) on the deposition date of January 30, 2014 with accession number of "NITE P-01797". Additionally, Novosphingobium sp. MBES04 was transferred from national deposit to international deposit in the NITE Patent Microorganism Depositary Center on the deposition date of February 9, 2015 (accession number: NITE ABP-01797).

As described above, microorganisms that can be used for the purpose of the present invention can be isolated from microorganisms existing in woods or the like where corrosion and rottenness are observed, by using the mycological properties, the physiological properties and the utilization potential as substrate and/or the base sequence of MBESS04 strain as indexes.

While there are no particular limitations to the method of culturing microorganisms of MBES04 strain, examples of culture methods that can be used for the purpose of the present invention include liquid culture methods (shaking culture method and aeration and agitation culture method) and preferably an aerobic liquid culture method is employed. From the industrial point of view, the use of an aeration and agitation culture method is more preferable. Any culture time may be used for the purpose of the present invention so long as microorganisms of MBES04 strain starts growing within the selected culture time. The culture time is, for example, between 8 and 120 hours. There are no particular limitations to the method of confirming the growth of microorganisms of MBES04 strain. For example, a culture specimen may be taken and observed through a microscope or observed for light absorption. Additionally, there are no particular limitations to the dissolved oxygen concentration of the culture solution but it is typically between 0.5 and 20 ppm. For the purpose of maintaining aerobic culturing conditions, the aeration rate needs to be regulated and the culture solution needs to be agitated, while oxygen may need to be added to the air to be used for aeration. Batch culture, fed-batch culture, continuous culture or perfusion culture may be used as culturing method for the purpose of the present invention.

Microorganisms to which any known microorganism utilization technique is applicable can be used for the purpose of the present invention. More specifically, microorganisms in various modes of existence may be used. Such microorganisms include not only microorganisms existing in liquid but also microorganisms in a state of being adsorbed or buried in a carrier and microorganisms in a freeze-dried state. Microorganisms that can be used for the purpose of the present invention may be a component of a kit for producing one or more than one phenyl propane-based compounds. When biomass is packaged with a solvent suitable for extracting lignins and one or more than one culture medium ingredients, it is possible to determine if the obtained biomass can be used as starting material for producing phenyl propane-based compounds.

Now, the present invention will be described in greater detail below by way of examples. It should be noted, however, that the present invention is by no means limited to the examples that are described below and the present invention can be carried out in various different modes so long as the problem to be solved by the present invention can actually be solved in any of such modes.

### [Examples]

### [Example 1. Screening of Lignin-Metabolizing Microorganisms]

A mixture obtained by suspending 0.2%W/V of sawdust of Japanese beech and 2%W/V of BACTO Agar in artificial sea water was treated in an autoclave at 121°C for 15 minutes. After the autoclave treatment, the mixture was cooled to 60°C and then IMK culture medium (available from Daigo) was added according to the procedure manual annexed to the product and supplied by the manufacturer. After adding the IMK culture medium, the mixture was solidified in a petri dish to turn it into a solid culture medium for isolating microorganisms.

A sunken coniferous tree that was eaten away by shipworms and well rotten was collected from the 260-m deep bottom of Suruga Bay. A 0.1 g sample of the sunken tree was powdered and suspended in artificial sea water (available from Daigo) and several drops of the suspension were applied onto the solid culture medium for isolating microorganisms. Then, the solid culture medium was held warm at 25°C for 10 days. Thereafter, the microorganisms that had formed colonies on the solid culture medium were transferred onto Difco Marine Agar 2216 (available from Becton Dickinson) and repeatedly subjected to streak culture at 25°C to obtain about 1,000 strains of microorganisms of a single genus.

A culture medium was prepared by mixing Tripticase Soy Broth (available from Becton Dickinson) and Difco Marine Broth 2216 culture medium (available from Becton Dickinson) at a ratio of 1:1 and guaiacylglycerol-*β*-guaiacyl ether (Compound I) was added to the culture medium so as to obtain a final concentration of 1 mM. In this way, a Compound I-containing culture medium was prepared. The isolated microorganisms were inoculated into the Compound I-containing culture medium. After culturing the microorganisms at 25°C and 120 rpm for 3 days, culture supernatant was obtained by means of a centrifugal operation conducted at 4,800 rpm for 5 minutes. 0.9 mL of methanol was added to 0.1 mL of the culture supernatant and mixed. Then, the mixture was subjected to a centrifugal operation conducted at 4,800 rpm for 5 minutes. The obtained culture supernatant was subjected to a reverse phase HPLC analysis under the following conditions. A decrease of Compound I was detected as a result of disappearance of a peak around the retention time of 5.7 minutes on the basis of a chromatogram showing absorbance at UV 270 nm.

### Reverse Phase HPLC Conditions

Column; Xbridge OST C18 (available from WATERS), 4.6 mm I.d. × 100 mm L. eluents; (A) [2 mM of ammonium acetate, 0.05%V/V formic acid], (B) methanol; solution feeding rate; 0-1 minutes 10%V/V (B), 1-8 minutes 10%V/V(B)-90%V/V (B), column temperature; 40°C, flow rate: 1.2 ml/min, detection; Photodiode Array Detector UV 200-500 nm (PDA model 2998, available from WATERS)
As a result of a reverse phase chromatography analysis of the culture supernatant, it was found that Compound I had completely disappeared from the culture supernatant inoculated with the microorganism strain named as MBES04 strain. As a result, it was found that the MBES04 strain was that of microorganisms having a potential ability of utilizing lignins and lignin-related substances.

### [Example 2. Identification of Metabolites Due to Degradation of Compound I by MBES04 Strain]

A Compound I-containing culture medium was prepared by adding Compound I to a medium produced by mixing Tripticase Soy Broth (available from Becton Dickinson) and Difco Marine Broth 2216 culture medium (available from Becton Dickinson) at a ratio of 1:1 so as to obtain a final concentration of 3 mM of Compound I and microorganisms of MBES04 strain were inoculated into the Compound I-containing culture medium. Part of the culture solution was extracted every 6 hours after the inoculation of microorganisms of MBES04 strain and the culture supernatant obtained by means of a centrifugal operation was subjected to a reverse phase HPLC analysis each time. As a result, it was found that, as Compound I decreases, new compounds are produced at retention time of about 4.4 minutes and at retention time of about 5.5 minutes respectively (see FIG. 1). These compounds are respectively named as Compound III and Compound VI. Compound VI was identified as guaiacol (Guaiacol in FIG. 2) on the basis of the retention time and the absorption spectrum thereof. It was confirmed that Compound III had been accumulated in the culture medium without being subjected to any additional metabolic transformation.

In order to identify Compound III, the above culture supernatant was refined by means of a solid phase extraction method (OASIS WAX; available from WATERS) and subjected to reverse phase ultraperformance liquid chromatography (UPLC)-time-of-flight precision mass spectrometry (ACCUITY UPLC H-Class, XevoG2 QTOF, available form WATERS) under the following conditions.

### Reverse Phase UPLC Conditions (UPLC-Time-of Flight Precision Mass Spectrometry)

Column; ACQUITY UPLC BEH C18 Column, 130 angstroms, 1.7 µm, (available from WATERS), 2.1 mm I.d. × 100 mm L (Part Number: 186002352) .
eluent; (A) [2 mM of ammonium acetate, 0.05%V/V formic acid], (B) [95%V/V acetonitrile], solution feeding rate; 0-5 minutes 5%V/V-95%V/V(B), 5-7 minutes 95%V/V(B), column temperature; 40°C. flow rate; 0.4 ml/min.
Mass Spectrometry Conditions (UPLC-time-of Flight Precision Mass Spectrometry)

Detection mass range 100-1,000 Da, data acquisition scanning interval 0.1 seconds, desolvation gas temperature 500°C, ion source ESI negative mode ion source temperature 150°C, cone voltage 20 V.

As a result of the reverse phase UPLC-time-of-flight precision mass spectrometry, m/z195.1 ions were detected from Compound III, and the molecular weight of Compound III was 196.1 and the compositional formula of Compound III was estimated to be C₁₀H₁₂O₄.

Substantially, Compound III was extracted three times from the microorganism culture supernatant by means of 100mL of ethyl acetate. The ethyl acetate layer was condensed, dried and solidified under reduced pressure to obtain 0.5 g of a crude product. The obtained crude product was refined by column chromatography in a manner as described below and 0.14 g of refined crystal was obtained from the fraction containing Compound III by evaporating the eluent solvent.

Column: silica gel (WAKO gel 200, available from WAKO Pure Chemical Industries) 2.1 cm I.d. × 110 mm L
Eluent: ethyl acetate/toluene = 2/3
Dispersed Compound III was dissolved in deuterochloroform and subjected to a spectrum obtaining operation using a 400 MHz nuclear magnetic resonance apparatus to obtain 1H-NMR, 13C-NMR spectrum.

The chemical shift values obtained by an NMR analysis of refined Compound III are listed below.
1H-NMR δ (ppm) : 3.19 (t, J=4.4, 2H, β-H), 3.96 (s, 3H, O-CH₃), 4.02 (t, J=4.4, 2H, γ-H), 6.12 (s, 1H, phenol-OH), 6.97 (d, J=6.8 1H, Ar-H), 7.55(m, 2H, Ar-H). 13C-NMR δ (ppm): 39.753 (β-C), 56.086 (OCH₃-C), 58.333 (γ-C), 109.549, 113.927, 123.660, 129.658, 146.677, 150.744 (Ar-C), 199.096 (α-C).

From the results of the above described analyses, Compound III was identified as 3-hydroxy-1-(4-hydroxy-3-methoxyphenyl)-1-propanone and it was found that a compound having a phenyl propane structure had been obtained.

### [Example 3. Identification of MBES04 Strain]

A culture medium was prepared by mixing Tripticase Soy Broth (available from Becton Dickinson) and Difco Marine Broth 2216 culture medium (available from Becton Dickinson) at a ratio of 1:1. Then, microorganisms of MBES04 strain were cultured on the prepared culture medium at 25°C and 120 rpm for 3 days and then subjected to a centrifugal operation at 4,800 rpm for 5 minutes to obtain cells of MBES04 strain. Then, all the DNAs were extracted from the cells of MBES04 strain by means of the DNA extraction kit NucleoSpin (registered trademark) Plant II (available from TAKARA BIO). A PCR was made to take place by using all the DNAs as template and also using primer 27f (5'-AGAGTTTGATCCTGGCTCAG-3') (sequence number 1) and 1525r (5'-AAAGGAGGTGATCCAGCC-3') (sequence number 2) and the base sequence of the obtained amplified fragment was analyzed. The conditions used for the PCR and those used for the base sequence analysis are as follow.

### PCR Conditions

1 × PCR buffer (containing MgCl₂) : 200 µm dNTPs, 0.6 µm 27f, 0.6 µm 1525r, 1.4 U of LA Taq DNA (Polymerase available from TAKATA BIO) Thermal Cycler temperature condition 97°C 2 minutes, [97°C 30 seconds, 60°C 1 minute, 72°C 90 seconds] × 30 cycles, 72°C 5 minutes

### Base Sequence Analysis

BigDye (registered trademark) Terminator v3.1 Cycle Sequencing Kit, v3.1 (available from Applied Biosystems) ABI 3730 XL DNA Analyzer (available from Applied Biosystems)
A BLAST sequence retrieval operation was conducted on the base sequence obtained by the above described base sequence analysis (DNA base sequence for encoding 16S ribosomal RNA) (sequence number 3) (Accession Number: AB733576) relative to NCBI nucleotide database16S ribosomal RNA sequences (Bacteria and Archaea) (http://www.ncbi.nlm.nih.gov/blast) by means of the Megablast (Optimize for highly similar sequences) method to find that microorganisms of MBES04 strain are those that belong to the genus Novosphingobium.

Additionally, it was found that the species that are closest to MBES04 strain are Novosphingobium pentaromativorans (Accession Number NR_041046) and Novosphingobium panipatense (Accession Number NR_044210). However, since the degree of agreement (identity) of 16S ribosomal RNA-DNA was 97%, microorganisms of MBES04 strain were determined to be those of a new species belonging to the genus Novosphingobium. Thus, the inventors of the present invention named the MBES04 strain as Novosphingobium species MBES04 and a conservation sample thereof was deposited in the Fermentation Res. Inst. Microorganism Depository Center (T̅292-0818 2-5-8 Kazusakamatari, Kisarazu City, Chiba Prefecture) of the National Institute of Technology and Evaluation (NITE) on the deposition date of January 30, 2014 with identification name of "Novosphingobium sp. MBES04" and accession number of "NITE P-01797".

### [Example 4. Investigation of Properties of MBES04 Strain]

Microorganisms of MBES04 strain could be cultured under the following conditions.
culture medium: liquid nourishment/solid culture medium containing MgSO₄ by 0.1%W/V
temperature: 10-45°C (optimum temperature 30°C)
pH: 5.5-8.5 (optimum pH6)
NaCl concentration: 0-6%W/V (optimum concentration 1%W/V)

The mycological properties of microorganisms of MBES04 strain are as follow.
(1) Morphological property
   bacillus of 0.3 to 0.8 × 2 to 3 µm
(2) Cultural properties

Microorganisms of MBES04 strain form circular colonies of a diameter of between 0.5 and 1.5 mm on an agar plate culture medium (0.1%W/V MgSO₄-added LB culture medium); at 30°C in 48 hours. All the peripheral edges of the colonies were smooth, lustrous and yellowy and showed a lowly convex profile.

As for the physiological properties of microorganisms of MBES04 strain, the results obtained by a physiological property test using an APIZYME are shown in Table 1 below. In Table 1, + indicates an instance where enzyme activity was observed and - indicates an instance where no enzyme activity was observed.

**[Table 1]**

| enzyme | activity |
|---|---|
| alkaline phosphatase | + |
| esterase (C4) | - |
| esterase lipase (C8) | - |
| lipase (C14) | + |
| leucine arylamidase | + |
| valine arylamidase | + |
| cystine arylamidase | + |
| trypsin | - |
| α-chymotrypsin | - |
| acid phosphatase | + |
| naphthol-AS-BI-phosphohydrolase | + |
| α-galactosidase | - |
| β-galactosidase | + |
| β-glucoronydase | + |
| α-glucosidase | - |
| β-glucosidase | + |
| N-acetyl-β-glucosaminidase | - |
| α-mannosidase | - |
| α-fucosidase | - |

Table 2 below shows the results obtained from a test on the utilization potential ability of MBES04 strain as substrate by using a GN2 plate (available from BIOLOG). In Table 2, + indicates an instance where the ability of utilization is observed and + indicates an instance where the ability of utilization is not observed.

**[Table 2]**

| | | | | | | | | | | | |
|---|---|---|---|---|---|---|---|---|---|---|---|
| | α-Cyclodextrin (-) | *β-*Cyclodextrin (+) | Dextrin (+) | Glycogen (-) | Inulin (-) | Mannan (-) | Tween 40 (-) | Tween 80 (-) | N-Acetyl-D-Glucosamine (+) | N-Acetyl-D-Mannosamine (-) | Amygdalin (+) |
| L-Arabinose (+) | D-Arabitol (+) | Arbutin (-) | D-Cellobiose (+) | D-Fructose (-) | L-Fucose (+) | D-Galactose (-) | D-Galacturonic acid (-) | Gentiobiose (-) | D-Gluconic acid (+) | *α*-D-Glucose (+) | m-Inositol (+) |
| α-D-Lactose (+) | Lactulose (+) | Maltose (+) | Maltotriose (-) | D-Mannitol (-) | D-Mannose (-) | D-Melezitose (-) | D-Melibiose (+) | *α-*MethylD-galactoside (+) | *β*-Methyl D-galactoside (-) | 3-Methylglucose (+) | *α*-Methyl D-glucoside (-) |
| *β-*MethylD-glucoside (+) | *α-*MethylD-mannoside (+) | Palatinose (-) | D-Psicose (-) | D-Raffinose (-) | L-Rhamnose (-) | D-Ribose (-) | Salicin (-) | Sedoheptulosan (+) | D-Sorbitol (-) | Stachyose (+) | Sucrose (-) |
| D-Tagatose (-) | D-Trehalose (-) | Turanose (+) | Xylitol (+) | D-Xylose (+) | Acetic acid (-) | *α-*Hydroxybutyric acid (-) | *β-*Hydroxybutyric acid (+) | γ-Hydroxybutyric acid (-) | ρ-Hydroxyphenyl acetic acid (-) | α-Keto Glutaric acid (-) | *α*-Keto valericacid (-) |
| Lactamide (+) | D-Lactic acid methyl ester (+) | L-Lactic acid (-) | D-Matic acid (+) | L-Malic acid (-) | Methyl pyruvate (+) | Monomethyl succinate (+) | Propionic acid (+) | Pyruvic acid (+) | Succinamic acid (+) | Succinic acid (+) | N-Acetyl L-glutamic acid (+) |
| L-Alaninamide (-) | D-Alanine (+) | L-Alanine (-) | L-Alanyl-glycine (-) | L-Asparagine (-) | L-Glutamic acid (-) | Glycyl- L-glutamic Acid (+) | L-Pyroglutamic acid (-) | L-Serine (+) | Putrescine (+) | 2,3-Butanediol (+) | Glycerol (-) |
| Adenosine (-) | 2-Deoxyadenosine (+) | Inosine (-) | Thymidine (-) | Uridine (-) | Adenosine-5'-monophosphate (+) | Thymidine-5'-monophosphate (+) | Uridine-5'-monophosphate (-) | Fructose-6-phosphate (+) | Glucose-1-phosphate (+) | Glucose-6-phosphate (+) | D-L-*α-*Glycerolphosphate (+) |

The properties of Novosphingobium pentaromativorans and those of Novosphingobium panipatense as described in Non-Patent Literature "Internal J Syst Evol Microbiol. 2009 Jan; 59 (Pt 1) : 156-61 Gupta SK et al. and those of MBES04 strain were compared. Table 3 shows the results of the comparison. From the results of the investigation on the properties of microorganisms of MBES04 strain, it was found that MBES04 strain is a strain of microorganisms that is different from Novosphingobium pentaromativorans and Novosphingobium panipatense in terms of that MBES04 strain has at least a potential ability of utilizing xylose.

**[Table 3]**

| | MBES04 | N. panipatense SM16^{T (+)} | N. pentaromativorans DSM 17173^{**T**(+)} |
|---|---|---|---|
| Growth at/in: | | | |
| 4° C | - | - | + |
| 41 ° C | + | ++ | + |
| 5% NaCl | + | - | + |
| Assimilation of: | | | |
| Sucrose | - | (+) | + |
| Malonate | + | - | (+) |
| Mannose | - | + | - |
| Mannitol | - | - | - |
| Arabinose | + | + | + |
| Fructose | - | + | - |
| Galactose | - | + | - |
| Rhamnose | - | - | + |
| Trehalose | - | + | + |
| Xylose | + | - | - |
| Sorbitol | - | + | - |
| Glycerol | - | - | - |
| Serine | + | + | - |

| | | | |
|---|---|---|---|
| **++ strongly positive** **+ positive** **(+) weekly positive** - **negative** **(***) **Data from Gupta SK et al.** Int J Syst Evol Microbiol. 2009 Jan:59(Pt 1):156-61**.** | | | |

### [Example 5. Production of Compounds Having a Phenyl Propane Structure from Glass Plant-Based Biomass]

Compounds having a phenyl propane structure were produced from extract solution of rice straws by following the procedures described below.

50 g of dried and powdered rice straw was suspended in a mixture solution of 300 mL of dioxane, which is widely recognized as solvent suitable for extracting lignins, and 15 mL of water and left in an immersed state at room temperature for 6 days. After the immersion treatment, the suspension was subjected to an operation of filtering the solid content of the suspension by means of filter paper to obtain a filtrate. After drying and solidifying the filtrate under reduced pressure by means of an evaporator, the evaporation residue was dried by means of a desiccator to obtain 2.65 g of solid. Then, the solid was dissolved by adding 50 ml of ethyl acetate and 70 mL of pure water to the solid. Thereafter, the ethyl acetate layer was extracted and dried under reduced pressure by means of an evaporator to obtain 0.86 g of solid. The solid was then dissolved in DMF so as to make it show a concentration of 10%W/V to obtain lignin-containing rice straw extract solution.

Difco Yeast Extract (available from Becton Dickinson) and MgSO₄ (available from WAKO Pure Chemical Industries) were added to ion exchange water so as to make them respectively show final concentrations of 0.5%W/V and 0.1%W/V and subsequently a small amount of 1N NaOH was dropped in the solution so as to make the solution show a pH value of 6.5. Then, the solution was filtered and sterilized by means of a 0.22 µM filter. The above described lignin-containing rice straw extract solution was aseptically added to the obtained filtrate by 1/40 capacity to produce an aseptic culture medium. The dioxin extract concentration in the aseptic culture medium was 2.5 mg/mL. The aseptic culture medium was subjected to a reverse phase UPLC-time-of-flight precision mass spectrometry under the conditions described in Example 2 but no guaiacylglycerol-*β*-guaiacyl ether (Compound I) was detected.

Microorganisms of MBES04 strain were inoculated into the obtained aseptic culture medium and cultured by shaking the culture medium at 30°C for 48 hours. Subsequently, the culture solution was subjected to a centrifugal operation at 10, 000 rpm for 5 minutes to obtain culture supernatant. 0.5 mL of the obtained culture supernatant was refined by means of a solid phase extraction method (OASIS WAX; available from WATERS) and then dried and solidified in nitrogen gas. After the drying and solidifying process, the residue was dissolved in 0.5 mL of 20%V/V acetonitrile and subjected to reverse phase UPLC-time-of-flight precision mass spectrometry under the conditions described in Example 2. As a control, a sample culture medium that had not been inoculated with microorganisms of MBES04 strain was processed in the same way and analyzed.

As a result of the reverse phase UPLC-time-of-flight precision mass spectrometry, it was confirmed that Compound III had been produced by 1.10 µg/mL due to the action exerted by the microorganisms of MBES04 strain. In the instance where no microorganisms of MBES04 strain had been inoculated, the comparable figure was 0.02 µg/mL. The yield was 0.04%W/W relative to the fractionated dry solid that is soluble to rice straw-containing dioxane ethyl acetate. As a result of the analysis, ions (225.1 m/z) of a compound having a molecular weight greater than that of Compound III by about 30 were detected. It corresponds to a compound having an additional methoxy group if compared with Compound III. By calculations on the basis of the calibration curve (without sensitivity correction) of 3-hydroxy-1-(4-hydroxy-3-methoxyphenyl)-1-propanone, the concentration of the compound was determined to correspond to 0.08 µg/mL from the peak area value of the mass chromatogram of the compound. Additionally, ions (165.1 m/z) of a compound having a molecular weight smaller than that of Compound III by about 30 were also detected. It corresponds to a compound that is short of a methoxy group if compared with Compound III. By calculations on the basis of the calibration curve (without sensitivity correction) of 3-hydroxy-1-(4-hydroxy-3-methoxyphenyl)-1-propanone, the concentration of the compound was determined to correspond to 0.73 µg/mL from the peak area value of the mass chromatogram of the compound. In the case of the sample where no microorganisms of MBES04 strain were inoculated, the concentration of Compound III and its derivatives was not greater than 0.01 µg/mL.

### [Example 6. Production of Compounds Having a Phenyl Propane Structure from Wood-Based Biomass]

Compounds having a phenyl propane structure were produced from oak sawdust extract solution by following the procedures described below.

20 g of dried and powdered oak sawdust was suspended in a mixture solution of 100 mL of dioxane and 5 mL of water and held in an immersed state at room temperature for 5 days. After the immersion, the solid fraction was removed by filtering the solution by means of filter paper to obtain a filtrate.

The obtained filtrate was dried by means of an evaporator under reduced pressure to obtain 0.53 g of an oak dioxane extract. The oak dioxane extract was then dissolved in 10%W/V of DMF. Difco Yeast Extract (available from Becton Dickinson) and MgSO₄ (available from WAKO Pure Chemical Industries) were added to ion exchange water so as to make them respectively show final concentrations of 0.5%W/V and 0.1%W/V and subsequently a small amount of 1N NaOH was dropped in the solution so as to make the solution show a pH value of 6.5. Then, the solution was filtered and sterilized by means of a 0.22 µM filter. The DMF solution of the above-described oak dioxane extract was aseptically added to the obtained filtrate by 1/40 capacity to produce an aseptic culture medium. The oak dioxane extract concentration in the aseptic culture medium was 2.5 mg/mL. The aseptic culture medium was subjected to reverse phase UPLC-time-of-flight precision mass spectrometry under the above described conditions but no guaiacylglycerol-*β*-guaiacyl ether (Compound I) was detected.

Microorganisms of MBES04 strain were inoculated into the obtained aseptic culture medium and cultured by shaking the culture medium at 30°C for 48 hours. Subsequently, the culture solution was subjected to a centrifugal operation at 10, 000 rpm for 5 minutes to obtain culture supernatant. 0.5 mL of the obtained culture supernatant was refined by means of a solid phase extraction method (OASIS WAX; available from WATERS) and then dried and solidified in nitrogen gas. After the drying and solidifying process, the residue was dissolved in 0.5 mL of 20%V/V acetonitrile and then subjected to reverse phase UPLC-time-of-flight precision mass spectrometry under the above described conditions. As a control, a sample culture medium that had not been inoculated with microorganisms of MBES04 strain was processed in the same way and analyzed.

As a result of the reverse phase UPLC-time-of-flight precision mass spectrometry, it was confirmed that Compound III had been produced by 0.93 µg/mL due to the action exerted by the microorganisms of MBES04 strain. In the instance where no microorganisms of MBES04 strain had been inoculated, the comparable figure was 0.02 µg/mL. The yield was 0.04%W/W relative to the oak dioxane extract. As a result of the analysis, ions (225.1 m/z) of a compound having a molecular weight greater than that of Compound III by about 30 were detected. It corresponds to a compound having an additional methoxy group if compared with Compound III. By calculations, the concentration of the compound was determined to correspond to 0.30 µg/mL from the peak area value of the mass chromatogram of the compound. Additionally, as a result of the reverse phase UPLC-time-of-flight precision mass spectrometry, ions (165.1m/z) of a compound having a molecular weight smaller than that of Compound III by about 30 were also detected due to the action exerted by the microorganisms of MBES04 strain. By calculations on the basis of the calibration curve (without sensitivity correction) of 3-hydroxy-1-(4-hydroxy-3-methoxyphenyl)-1-propanone, it was confirmed that the compound had been produced to a concentration corresponding to 0.90 µg/mL from the peak area value of the mass chromatogram of the compound.

### [Example 7. Production of Compounds Having a Phenyl Propane Structure from Agricultural Waste-Based Biomass]

Compounds having a phenyl propane structure were produced from a waste Shiitake mushroom bed by following the procedures described below.

10 g of a dried and powdered waste Shiitake mushroom bed was suspended in ion exchange water so as to make it show a concentration of 10%W/V and held in a warmed condition at 60°C in a water bath for 3 hours. After the end of the warmed condition, the solid fraction was removed from the suspension by filtering using filter paper to obtain a filtrate. The weight of the dried and removed solid was 1.88 g. As a result of subjecting the filtrate to UPLC-time-of-flight precision mass spectrometry, guaiacylglycerol-*β*-guaiacyl ether (Compound I) was not detected from the filtrate.

1N NaOH was dropped into the obtained filtrate by a small amount so as to adjust the pH of the filtrate to 6.5 and subsequently Difco Yeast Extract (available from Becton Dickinson) and then MgSO₄ (available from WAKO Pure Chemical Industries) were added to the filtrate so as to make them respectively show final concentrations of 0.5%W/V and 0.1%W/V. The obtained solution was filtered and sterilized by means of a 0.22 µM filter to produce an aseptic culture medium. Then, microorganisms of MBES04 strain were inoculated into the aseptic culture medium. After culturing the microorganisms at 30°C for 72 hours, the culture solution was subjected to a centrifugal operation at 10,000 rpm for 5 minutes to obtain culture supernatant. Then, 0.5 mL of the obtained culture supernatant was refined by means of a solid phase extraction method (OASIS WAX; available from WATERS) and then dried and solidified in nitrogen gas. After the drying and solidifying process, the residue of the drying and solidifying process was dissolved in 0.5 mL of 20%V/V acetonitrile and subjected to reverse phase UPLC-time-of-flight precision mass spectrometry under the conditions described in Example 2.

As a result of the reverse phase UPLC-time-of-flight precision mass spectrometry, it was confirmed that Compound III had been produced by 2.96 µg/mL due to the action exerted by the microorganisms of MBES04 strain. In an instance where no microorganisms of MBES04 strain had been inoculated, the comparable figure was 0.09 µg/mL. The yield was 0.02%W/W relative to the hot water extract of the waste Shiitake mushroom bed. As a result of the analysis, ions (225.1 m/z) of a compound having a molecular weight greater than that of Compound III by about 30 were detected. By calculations on the basis of the calibration curve (without sensitivity correction) of 3-hydroxy-1-(4-hydroxy-3-methoxyphenyl)-1-propanone, the concentration of the produced compound was determined to correspond to 1.61 µg/mL from the peak area value of the mass chromatogram of the compound. Additionally, it was confirmed that ions (165.1 m/z) of a compound having a molecular weight smaller than that of Compound III by about 30 had been produced. By calculations, it was confirmed that the compound had been produced to a concentration corresponding to 5.89 µg/mL.

### [Example 8. Measurement of Production Efficiency of 3-hydroxy-1-(4-hydroxy-3-methoxyphenyl)-1-propanone by Means of Microorganisms of MBES04 Strain]

Microorganisms of MBES04 strain were inoculated into 120 mL of a culture medium containing 1M of guaiacylglycerol-*β*-guaiacyl ether (Compound I, to be abbreviated as GGGE hereinafter) and 5 mM of MgSO₄ LB culture medium and cultured by shaking the culture medium at 30°C. 2 mL of the culture solution was extracted every 6 hours from the start of the culturing process to 48 hours after the start and every 24 hours from 48 hours after the start, and subjected to a centrifugal operation at 14, 000 rpm for 5 minutes to obtain culture supernatant. Then, 9-hydroxyfluoren was added to the culture supernatant as internal standard material so as to make it show a final concentration of 0.025 mM and the supernatant was diluted to 10-fold dilution by methanol. Then, the diluted supernatant was subjected to a centrifugal operation at 14, 000 rpm for 5 minutes to remove the impurities. The obtained supernatant was then subjected to an HPLC analysis under the conditions same as those described in Example 1 and the decrease of Compound I and the newly produced metabolite were quantified. The values obtained by the quantifications were corrected on the basis of the peak area value of the internal standard material in order to eliminate the influence of the errors, if any, that had been involved in the analysis process. The production ratio was determined by computations by referring to the ratio when each of the components was collected with the concentration same as the concentration at the time of initial addition of 1 mM, which was defined as 100%. As a result, it was found that Compound III could be collected with a yield of not less than 50% after the elapse of 30 hours. Even when the culture time was extended to 120 hours, Compound III did not decrease and could be collected from the culture solution of MBES04 strain at a yield not less than 50% (see FIG. 1). FIG. 2 shows the chromatogram obtained by using the samples obtained in 48 hours from the start.
[Sequence Number 1] Primer 27f
   AGAGTTTGATCCTGGCTCAG
[Sequence Number 2] Primer 1525r
   AAAGGAGGTGATCCAGCC
[Sequence Number 3] 16S ribosomal RNA-DNA of MBES04 strain

### [Industrial Applicability]

3-hydroxy-1-(4-hydroxy-3-methoxyphenyl)-1-propanone can be obtained from biomass containing natural lignins by means of the production method of the present invention and microorganisms provided herein. 3-hydroxy-1-(4-hydroxy-3-methoxyphenyl)-1-propanone can be transformed into various industrially useful compounds and also can be used as starting material for producing 1-(4-hydroxy-3-methoxyphenyl)-1,3-propanediol and so on, which can be utilized as starting material for producing resins, adhesive agents, resist materials and drugs among others.

## Claims

1. A method for producing a phenyl propane-based compound comprising a step of producing a phenyl propane-based compound
expressed by general formula (A) 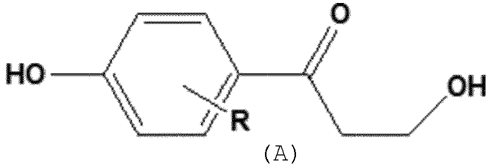 wherein R represents
- 1, 2 or more than 2 alkyl groups or alkoxy groups having 1 to 5 carbon atoms or
- hydrogen atoms
by causing the microorganism Novosphingobium species MBES04 (accession number: NITE P-01797) to act on biomass containing lignins.

2. The method according to claim 1, wherein the phenyl propane-based compound is at least one selected from a group of 3-hydroxy-1-(4-hydroxy-3-methoxyphenyl)-1-propanone, 3-hydroxy-1-(4-hydroxy-3,5-dimethoxyphenyl)-1-propanone and 3-hydroxy-1-(4-hydroxyphenyl)-1-propanone.

## Patentansprüche

1. Ein Verfahren zur Herstellung einer Verbindung auf Phenylpropanbasis, umfassend einen Schritt der Herstellung einer Verbindung auf Phenylpropanbasis, welche durch die allgemeine Formel (A) ausgedrückt ist 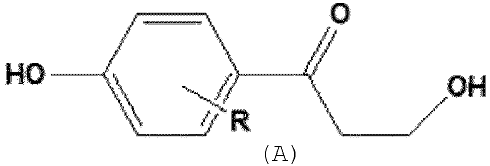 worin R
- - 1, 2 oder mehr als 2 Alkylgruppen oder Alkoxygruppen mit 1 bis 5 Kohlenstoffatomen oder
- - Wasserstoffatome
repräsentiert,
indem man den Mikroorganismus Novosphingobium species MBES04 (Hinterlegungsnummer: NITE P-01797) auf ligninhaltige Biomasse einwirken läßt.

2. Das Verfahren gemäß Anspruch 1, wobei die Verbindung auf Phenylpropanbasis mindestens eine aus einer Gruppe von 3-Hydroxy-1-(4-hydroxy-3-methoxyphenyl)-1-propanon, 3-Hydroxy-1-(4-hydroxy-3,5-dimethoxyphenyl)-1-propanon und 3-Hydroxy-1-(4-hydroxyphenyl)-1-propanon ausgewählte ist.

## Revendications

1. Procédé de production d'un composé à base de phénylpropane comprenant une étape consistant à produire un composé à base de phénylpropane exprimé par la formule générale (A) 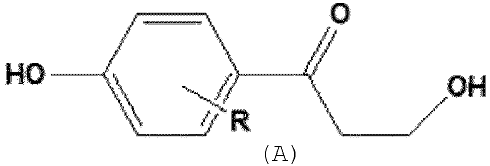 dans laquelle R représente
- 1, 2 ou plus de 2 groupes alkyle ou groupes alcoxy ayant 1 à 5 atomes de carbone ou
- des atomes d'hydrogène
en amenant le microorganisme Novosphingobium espèce MBES04 (numéro d'entrée : NITE P-01797) à agir sur de la biomasse contenant des lignines.

2. Procédé selon la revendication 1, dans lequel le composé à base de phénylpropane est au moins un sélectionné parmi un groupe de 3-hydroxy-1-(4-hydroxy-3-méthoxyphényl)-1-propanone, 3-hydroxy-1-(4-hydroxy-3,5-diméthoxyphenyl)-1-propanone et 3-hydroxy-1-(4-hydroxyphényl)-1-propanone.


### SEQUENCE LISTING

<110> Japan Agency for Marine-Earth Science and Technology (JAMSTEC)
<120> A method to produce phenylpropane compouds using a microorganism
<130> 15DF0302PCT
<160> 3
<170> PatentIn version 3.5
<210> 1
   <211> 20
   <212> DNA
   <213> Artificial sequence
<220>
   <223> Primer
<400> 1
   agagtttgat cctggctcag 20
<210> 2
   <211> 18
   <212> DNA
   <213> Artificial sequence
<220>
   <223> Primer
<400> 2
   aaaggaggtg atccagcc 18
<210> 3
   <211> 1442
   <212> DNA
   <213> Novosphingobium sp. MBES04
<400> 3
   aacgaacgct ggcggcatgc ctaacacatg caagtcgaac gaacccttcg gggttagtgg       60
   cgcacgggtg cgtaacacgt gggaatctgc ctcttggttc ggaataacag tgagaaatta     120
   ctgctaatac cggatgatga cttcggtcca aagatttatc gccaagagat gagcccgcgc     180
   aggattaggt agttggtggg gtaaaggcct accaagccga cgatccttag ctggtctgag     240
   aggatgatca gccacactgg gactgagaca cggcccagac tcctacggga ggcagcagtg     300
   gggaatattg gacaatgggc gaaagcctga tccagcaatg ccgcgtgagt gatgaaggcc     360
   ttagggttgt aaagctcttt taccagggat gataatgaca gtacctggag aataagctcc     420
   ggctaactcc gtgccagcag ccgcggtaat acggagggag ctagcgttgt tcggaattac     480
   tgggcgtaaa gcgcgcgtag gcggttactc aagtcagagg tgaaagcccg gggctcaacc     540
   ccggaactgc ctttgaaact aggtgactag aatcttggag aggtcagtgg aattccgagt     600
   gtagaggtga aattcgtaga tattcggaag aacaccagtg gcgaaggcga ctgactggac     660
   aagtattgac gctgaggtgc gaaagtgtgg ggagcaaaca ggattagata ccctggtagt     720
   ccacaccgta aacgatgata actagctgtc cgggttcttg gaatttgggt ggcgcagcta     780
   acgcattaag ttatccgcct ggggagtacg gtcgcaagat taaaactcaa aggaattgac     840
   gggggcctgc acaagcggtg gagcatgtgg tttaattcga agcaacgcgc agaaccttac     900
   cagcgtttga catcctcatc gcgatttcca gagatggatt tcttcagttc ggctggatga     960
   gtgacaggtg ctgcatggct gtcgtcagct cgtgtcgtga gatgttgggt taagtcccgc    1020
   aacgagcgca accctcatcc ttagttgcca gcatttagtt gggcactcta aggaaactgc    1080
   cggtgataag ccggaggaag gtggggatga cgtcaagtcc tcatggccct tacacgctgg    1140
   gctacacacg tgctacaatg gcggtgacag tgggcagcaa gtgcgcgagc acaagctaat    1200
   ctccaaaagc cgtctcagtt cggattgttc tctgcaactc gagagcatga aggcggaatc    1260
   gctagtaatc gcggatcagc atgccgcggt gaatacgttc ccaggccttg tacacaccgc    1320
   ccgtcacacc atgggagttg gtttcacccg aaggtagtgt gctaaccgca aggaggcagc    1380
   taaccacggt gggatcagcg actggggtga agtcgtaaca aggtagccgt aggggaacct    1440
   gc                                                                   1442